# EUROPEAN PATENT APPLICATION

(11) **EP 4 389 883 A1**
(43) Date of publication of application: **26.06.2024**
(21) Application number: 22216326.3
(22) Date of filing: 23.12.2022
(51) Int. Cl.: C12N 9/18, A61L 27/14, C08L 67/04, C12P 7/625, A61L 27/18, A61L 27/58, A61L 31/14, C08J 11/10, C12M 1/12

(54) **POLYMER SUBSTRATE-ENZYME COMPLEX**

(71) Applicant: Schwab, Helmut, 8043 Graz (AT)
(72) Inventor: Schwab, Helmut, 8043 Graz (AT)
(74) Representative: Schwarz & Partner Patentanwälte GmbH

(57) **Abstract**

The present invention relates a method for obtaining a storage stable polymer substrate-enzyme complex comprising:
a) providing a three-dimensional polymer structure comprising polyhydroxybutyrate (PHB) or a copolymer thereof,
b) contacting the three-dimensional polymer structure of step a) with at least one polyhydroxybutyrate degrading enzyme in a solvent to form a three-dimensional polymer structure-enzyme complex, and
c) reversibly decreasing the enzymatic activity of the least one polyhydroxybutyrate degrading enzyme.

## Description

### FIELD OF THE INVENTION

The present invention relates to polymers comprising polyhydroxybutyrate (PHB).

### BACKGROUND ART

Biodegradable polymers play an important role in various fields including packaging, tissue cultivation and medicine. For example, biodegradable polymers can be used as carrier or scaffold in cell and tissue cultivation or in medicine as implants or to coat implants and/or to control the release of pharmaceutically active compounds.

Biodegradable polymers can be of biosynthetic origin produced by living organisms or by bio-catalytic synthesis routes. Biodegradable polymers can also be obtained by chemical synthesis from monomeric units.

Biodegradable polymers can be degraded, for instance, by the action of enzymes. In case of packaging material comprising such polymers, polymer degrading enzymes are provided by microorganisms or other organisms which are contacted with the polymer upon release into the environment.

Implants or controlled release compositions may comprise biodegradable polymers which are degraded by enzymes naturally occurring in animals or humans, or in cultivated cells. This does not allow an appropriate control of the degradation process which is influenced mainly by the activity of the enzymes present in the respective tissues where such compositions are applied. This is usually mainly defined by the rate of production of the degrading enzymes. For some biodegradable polymers like polyhydroxybutyrate (PHB), degrading enzymes are not produced by animals or humans, resulting in no or only very slow degradation (e.g. by inefficient chemical degradation) of implants, scaffolds or release compositions.

In addition, these polymer degrading enzymes have been found to be unstable under various storage conditions. It is known that enzymes tend to lose enzymatic activity if stored in an aqueous environment.

It is an object of the present invention to provide compositions which comprise a storage stable biodegradable polymer which can be degraded, and which can be used as biodegradable implants, biodegradable carriers or scaffolds, or to controllably release pharmaceutically active ingredients in or on the animal or human body.

### SUMMARY OF THE INVENTION

Thus, the present invention relates to a method for obtaining a storage stable polymer substrate-enzyme complex comprising:
a) providing a three-dimensional polymer structure comprising polyhydroxybutyrate (PHB) or a copolymer thereof,
b) contacting the three-dimensional polymer structure of step a) with at least one polyhydroxybutyrate degrading enzyme in a solvent to form a three-dimensional polymer structure-enzyme complex, and
c) reversibly decreasing the enzymatic activity of the least one polyhydroxybutyrate degrading enzyme.

It turned surprisingly out that a three-dimensional polymer structure comprising polyhydroxybutyrate and comprising at least one polyhydroxybutyrate degrading enzyme attached to the surface of said polymer structure can be stored under various conditions conserving the enzymatic activity of the polyhydroxybutyrate degrading enzyme. Its enzymatic activity can be restored upon modifying the storage conditions. In particular, a polyhydroxybutyrate degrading enzyme can be contacted with the three-dimensional polymer structure allowing a controlled and continuous degradation of the polymer into its monomers and oligomers. Attempts to create an association of depolymerizing enzymes with polymers were performed by adding the enzyme after dissolving the polymer in an appropriate non-aqueous solvent (see for example Khan et al., ACS Omega 2019, 4, 2844-2852) or after melting it (see for example Huang et al., Biomacromolecules 2020, 21, 3301-3307). However, both strategies are quite limited as applications via dissolved polymers are not suited for many applications and enzymes may get inactivated by the high temperature treatment needed for melting the polymer.

Polyhydroxybutyrate degrading enzymes may contain a protein domain which supports the binding of the enzyme to the polymer. It turned surprisingly out that the activity of such binding domain containing enzymes can be reversibly decreased during the storage of the complex under various conditions and increased even after long time periods when bound to a polymer. The amount of bound enzyme, and thus the velocity of degradation can be significantly enhanced by increasing the surface area of the three-dimensional polymer structure by introducing structures to objects made up by a polymer. Such structures can be organized randomly or in a defined geometric manner and can be introduced simultaneously with the construction of polymer objects or introduced afterwards.

Another aspect of the present invention relates to a storage stable polymer substrate-enzyme complex obtained by said method. The polymer substrate-enzyme complex comprises a three-dimensional polymer structure and at least one polyhydroxybutyrate polymer degrading enzyme attached on the surface of said polymer. The complex of the present invention can be stored under various conditions where the enzyme activity is reversibly decreased, wherein controlled and continuous degradation of the polymer into its monomers and/or oligomers is initiated upon modifying the conditions suited for restoring the activity of the polymer degrading enzyme.

A further aspect of the present invention relates to the use of a storage stable polymer structure-enzyme complex as an or as a part of an implant and/or as carrier or scaffold in human and/or animal cell culture. It turned out that the polymer structure-enzyme complex of the present invention can be used as part of an implant and/or as carrier or scaffold in human and/or animal cell culture. The presence of the polyhydroxybutyrate polymer degrading enzyme results in a relatively slow degradation of PHB whereby tissue or human/animal cells surrounding the implant or the scaffold, respectively, take over the function of the implant or scaffold over the time.

### SHORT DESCRIPTION OF THE FIGURES

Fig. 1 shows the release of dye from PHB to the buffer by the action of a solid PHB depolymerase, liquid PHB depolymerase and the positive control without integrated degrading enzyme and 50 µL enzyme (0.2 mg/mL) added externally.
Fig. 2 shows the degradation of surface enhanced PHB sheets by PHB-DP expressed as release of D-3-hydroxybutyrate monomer (3-HB) dependent on reaction time. The negative control was PHB sheet incubated with 200 mM potassium phosphate buffer (no enzyme).
Fig. 3 shows a microscopic analysis of the sheets obtained from electrospinning of PHB. A, sheet #1, B, sheet #2, C sheet #3; magnification 100 x.
Fig. 4 shows treatment of sheets obtained by electrospinning (see Fig. 3) with PHB-DP.
Fig. 5 shows objects obtained by laser sintering.
Fig. 6 shows the release of 3-HB from laser sintered objects loaded with PHB-DP and stored for up to 14 days.
Fig. 7 shows PHB objects generated by laser burning. a, b and c show magnifications of the burned areas shaped as lamellar, pyramidal and cylindrical, respectively, forms. d shows an example showing the 10 x 10 mm area where the structures were burned.
Fig. 8 shows a model for creating a polymer object with enhanced surface. The cones with the "positive" unit (A) had a height of 2.5 mm, the conic holes of the "negative" unit (B) had a corresponding size in order to create a distance of 0.2 mm between the cones and the conic wholes. The "negative" unit in addition had an elevated outer ring of 0.5 mm. The diameter of the model was 50 mm.
Fig. 9 shows a three-dimensional polymer structure obtained by thermo-hydraulic pressing. Views from both sides are shown.
Fig. 10 shows the effect of long-time storage on activity of PHB-DP associated to PHB. A: storage at room temperature, B: storage at 4°C. As the particle size was not totally homogeneous, a higher variation is observed with the different samples taken at the different times.
Fig. 11 shows the release of iodine from polyvidone-iodine complex integrated in PHB by PHB-DP. Due to the small size of the analyzed sheets only small amounts of iodine were released. A: PHB sheet with integrated polyvidone-iodine complex; B: PHB sheet without polyvidone-iodine complex; C: Release of iodine measured.
Fig. 12 shows an SDS Polyacrylamide gel of supernatants of cultures of *Acidovorax spp.* BT293. Lane 1, size standard (page ruler, Thermofisher Scientific); Lanes 2 .4, cultivation at 37°C; lanes 5-7, cultivation at 28°C; lanes 2 & 5, no addition; lanes 3 & 6, addition of 0.1 g/L 3-HB; lanes 4 & 7, addition of 1 g/L PHB.

### EMBODIMENTS OF THE INVENTION

"Storage stable", as used herein, means that the enzymatic activity of the polymer degrading enzyme within the polymer substrate-enzyme complex is substantially preserved. "Substantially preserved", as used herein, means that the polymer degrading enzyme shows after decreasing and restoring the enzymatic activity at least 25%, preferably at least 30%, preferably at least 35%, preferably at least 40%, preferably at least 45%, preferably at least 50%, preferably at least 55%, preferably at least 60%, preferably at least 65%, preferably at least 75%, preferably at least 80%, preferably at least 85%, preferably at least 90%, preferably at least 95%, enzymatic activity compared to the enzymatic activity before decreasing enzymatic activity of the polymer degrading enzyme bound to the three-dimensional polymer structure. The term "decreasing", as used herein, refers to the reduction of the enzymatic activity and/or the rate of enzymatic reactions of the polymer degrading enzyme of at least 25%, preferably at least 30%, preferably at least 35%, preferably at least 40%, preferably at least 45%, preferably at least 50%, preferably at least 55%, preferably at least 60%, preferably at least 65%, preferably at least 75% , preferably at least 80%, preferably at least 85%, preferably at least 90%, preferably at least 95% compared to the enzymatic activity before decreasing the enzymatic activity of the polymer degrading enzyme. The enzymatic activity can be decreased by known methods in the art. "Reversibly decreasing", as used herein, means that the enzymatic activity of the polymer degrading enzyme can be restored after decreasing said enzymatic activity. Preferably, after the step of reversibly decreasing the enzymatic activity of the least one polyhydroxybutyrate degrading enzyme, the enzymatic activity of the enzyme within the polymer substrate-enzyme complex is substantially preserved, as defined above.

After reversibly decreasing the enzymatic activity of the at polymer degrading enzyme, the obtained complex can be stored under various condition such as for example, chilled, frozen, moist or dry conditions. Preferably, the complex is stored under dry conditions (less than 5% humidity, preferably less than 2% humidity, more preferably less than 1% humidity). In the following, the terms "storage" and "stored" refer to the storage of the polymer substrate-enzyme complex under chilled, frozen, moist or dry conditions, unless specified differently, over a period of at least 10 days, preferably at least six months, preferably at least 9 months, more preferably at least 12 months, at a temperature of about 20°C (room temperature) to 40°C or less (e.g. 4°C (fridge) or -20°C (freezer)) .

"Polymer substrate", as used herein, refers to a substance which comprises two or more subunits ("monomers") which are covalently attached to each other to form a branched or unbranched chain. Two or more of such chains may be crosslinked, i.e. covalently bound to each other. The monomers forming the polymer may be identical or structurally different. Polymer substrates comprising two or more of the same monomer are homopolymers, whereas polymers comprising two or more chemically distinct monomers are copolymers. Preferably, the polymer is polyhydroxybutyrate (PHB) or a copolymer thereof. According to another preferred embodiment of the present invention the polyhydroxybutyrate (PHB)is selected from the group consisting of poly-3-hydroxybutyrate and poly-4-hydroxybutyrate. According to a further preferred embodiment the copolymer can be poly(3-hydroxybutyrate-co-3-hydroxyvalerate).

"Polymer substrate-enzyme complex" as used herein, refers to an entity formed when at least one polymer substrate is contacted to at least one polymer degrading enzyme in a solvent. Preferably, the polymer degrading enzyme comprises a specific binding domain facilitating the attachment of the enzyme to the polymer. Preferably, the at least one polymer degrading enzyme is a polyhydroxybutyrate degrading enzyme. According to another preferred embodiment of the present invention the solvent is an aqueous solution and/or an organic solvent or a combination thereof.

"Three-dimensional polymer structure", as used herein, refers to a polymer substrate having measurements of at least 10 µm in three (x,y,z) directions respectively, wherein the vectors in these directions do not lie in the same plane. According to another preferred embodiment of the present invention the three-dimensional polymer structure has measurements of at least 50 pm, preferably of at least 100 pm, more preferably of at least 1000 µm in three (x,y,z) directions respectively, wherein the vectors in these directions do not lie in the same plane. According to another embodiment of the present invention, the three-dimensional polymer structure can have the form of a cube, a cuboid, a sphere, a cylinder, a prism, a pyramid, a cone or combinations thereof. Preferably, additional structures can be introduced within the three-dimensional polymer structure and/or on the surface of said three-dimensional polymer structure to enhance the specific surface area of said polymer. Such structures can be organized randomly or in a defined geometric manner and can be introduced simultaneously with the manufacturing of the three-dimensional polymer structure or introduced afterwards.

According to a further preferred embodiment of the present invention the three-dimensional polymer structure may be provided in a solid form.

"Polyhydroxybutyrate degrading enzymes", as used herein, are enzymes which are able to break any chemical bond between the subunits ("monomers") of a polyhydroxybutyrate polymer.

According to another preferred embodiment of the present invention, the polyhydroxybutyrate degrading enzyme can be provided in a solid state or in solution.

According to a further preferred embodiment of the present invention, the solid polyhydroxybutyrate degrading enzyme can be provided in lyophilized or spray dried form. Methods for producing such enzyme preparations are well known in the art.

In order to obtain a composition of the present invention the respective components may be solubilized using solvents, before contacting them.
In particular, three-dimensional polymer structures can be solubilized using organic solvents like chloroform, dichloromethane, dimethyl carbonate, fluorinated solutions (such as hexafluoro isopropanol) or aqueous solutions. The at least one polyhydroxybutyrate degrading enzyme can also be suspended in a solvent whereby it is particularly preferred that the aforementioned enzyme is suspended in the same solvent. The main components of the complex of the present invention, the three-dimensional polymer structure and the at least one polyhydroxybutyrate degrading enzyme, can be provided in solution or in a solid state, whereby it is particularly preferred to provide the polymer in a solid state and the enzyme in solution.

The three-dimensional polymer structure of the present invention can be produced by 3-D printing, electrospinning, laser sintering, thermo-mechanical structuring, and combinations thereof. In particular, the polymer is provided in the form of a powder and/or pellets and/or a filament before processing.

According to another preferred embodiment of the present invention, the specific surface area of said three-dimensional polymer structure can be increased by a chemical, physical, enzymatic method or a combination thereof. Preferably, pores, holes, folds, grooves, channels, wells, and combinations thereof can be formed within the three-dimensional polymer structure to increase the specific surface area of said polymer.

According to another preferred embodiment of the present invention the physical method to increase the specific surface area of the three-dimensional polymer structure is selected from the group consisting of milling, drilling, grinding, cutting, embossing, and/or the machining of structures from powders, filaments, pellets, tubes, and combinations thereof.

A further possibility to enhance the specific surface area of the three-dimensional polymer structure is creating a combination structure of a polymer with a further polymeric structure having a high surface area such as e.g. filters or textures (made of cellulose, wool or other polymeric substances) .

According to a further preferred embodiment of the present invention, the chemical or enzymatic method to increase the specific surface area of the three-dimensional polymer structure is selected from the group consisting of etching, partial degradation, partial hydrolysis, partial dissolving, partial breaking of polymer strands and combinations thereof. In particular, the surface area of the three-dimensional polymer structure can be increased by etching the surface of the polymer by applying at least one acid or base such as HCl, NaOH, H₂SO₄, NH₄OH a carboxylic acid or a combination thereof to said polymer. Partial hydrolysis of said polymer structures by the action of the at least one polyhydroxybutyrate degrading enzyme or partial dissolving by the action of a suited solvent such as for example chloroform or hexafluoro isopropanol or dimethyl carbonate can also be applied, also in combinations with the mentioned methods.

According to a further preferred embodiment of the present invention, the at least one polyhydroxybutyrate degrading enzyme may comprise a polyhydroxybutyrate (PHB) binding domain and is preferably selected from the group consisting of hydrolases, esterases, polyesterases, polyester depolymerases, polyhydroxyalkanoate depolymerase, polyhydroxybutyrate depolymerase and combinations thereof. The attachment of the enzyme to the polymer can be facilitated by compounds which act in a way that the enzyme sticks to the surface of the polymer (coating) or by using a polyhydroxybutyrate degrading enzymes containing a specific protein domain (binding domain) facilitating binding of the enzyme to the polymer. This has the advantage that upon the degradation process the enzyme molecules stay attached or may reattach to the remaining polymer molecules.

The at least one polyhydroxybutyrate degrading enzyme according to the present invention is able to degrade the three-dimensional polymer structure by cleaving chemical bonds between the monomers within the polymer chain and/or by cleaving chemical bonds crosslinking the chains of the polymer. The polyhydroybutyrate degrading enzyme be an enzyme catalyzing the hydrolysis from one end of the polymer chain (exo-enzyme) or catalyzing the cleavage of one or more linking ester bonds within the polymer (endo-enzyme).

According to a further preferred embodiment of the present invention, the at least one polyhydroxybutyrate degrading enzyme can be polyhydroxyalkanoate depolymerase, preferably a polyhydroxyalkanoate depolymerase derived from bacteria, archaea, or fungi. Polyhydroxyalkanoate depolymerases are a family of enzymes known to degrade polyhydroxyalkanoates (PHAs).

According to another preferred embodiment of the present invention the polyhydroxyalkanoate depolymerase is an extracellular polyhydroxyalkanoate depolymerase.

According to a further preferred embodiment of the present invention the polyhydroxyalkanoate depolymerase is an extracellular polyhydroxyalkanoate depolymerase derived preferably from bacteria, especially of the bacterial families *Burkholderiales* and *Comamonadaceae*, for example from *Acidovorax* species or *Burkholderiales* species.

According to another preferred embodiment of the present invention the at least one polyhydroxyalkanoate depolymerase is from *Acidovorax* species and comprises or consists of preferably of a sequence having the amino acid sequence WP_015014950, WP_056747090, WP_056064600, WP_056640205, WP 099730713 or WP_094287388 (NCBI database entry: http://www.ncbi.nlm.nih.gov/) or having an amino acid sequence being at least 80% identical to the abovementioned proteins.

According to another preferred embodiment of the present invention the at least one polyhydroxyalkanoate depolymerase can be obtained from *Burkholderiales* species and comprises or consists of preferably a sequence having the amino acid sequence OGA61346 (GenBank), OGB11797 (GenBank) or OGA86463 (GenBank).

A particular preferred extracellular polyhydroxyalkanoate depolymerase comprises or consists of the amino acid sequence SEQ ID No. 1 or SEQ ID No. 2 (processed form of SEQ ID No. 1).

In a further preferred embodiment of the present invention the polyhydroxyalkanoate depolymerase comprises preferably an affinity tag useful for rapid purification, which is preferably located at its C-terminal end, and is preferably a polyhistidine tag (e.g. His6-Tag).

According to a further preferred embodiment of the present invention, the at least one polyhydroxybutyrate degrading enzyme is a polyhydroxybutyrate depolymerase. Polyhydroxybutyrate depolymerase can act in a way to degrade PHB structures consisting of (*R*)-3-hydroxybutyrate monomers linked to polymers via ester bonds between the carboxyl and the hydroxyl groups. In particular, the polyhydroxybutyrate depolymerase can be contacted with the three-dimensional polymer structure consisting of PHB or a copolymer thereof allowing a controlled and continuous degradation of PHB into its (*R*)- 3-hydroxybutyrate monomers.

According to another preferred embodiment of the present invention the polyhydroxybutyrate depolymerase comprises an amino acid sequence having at least 80% identity to SEQ ID No. 2 and a motif comprising amino acid sequence ANLQXSKVYL (SEQ ID No. 4), wherein X is threonine, serine, glycine, glutamic acid, glutamine or aspartic acid. It turned out that the polyhydroxybutyrate depolymerase of the present invention exhibits an efficient enzymatic hydrolysis activity resulting in a superior PHB degradation compared to other enzymes comprising different amino acids at this position of said motif. Preferably, threonine at position X of the motif comprising amino acid sequence ANLQXSKVYL (SEQ ID No. 4) may have a significant effect on the activity of the inventive enzyme. Upon contacting the at least one polyhydroxybutyrate depolymerase with the polymer comprising PHB or a copolymer thereof, the monomeric (*R*)-3-hydroxybutyrate units of PHB are progressively cleaved off from one end of the polymer stands, resulting in enantiopure (*R*)-3-hydroxybutyrate monomers. 3-hydroxybutyrate is an important metabolite in living organisms, whereas only the (*R*)- enantiomer is present. It is of importance for food and pharmaceutical applications to provide the (*R*)-enantiomer, as the (*S*)- enantiomer does not have the intended function in biological systems and can have adverse effects on human and animal health if present in mixtures of (*R*)and (*S*) enantiomers. Hence, another advantage of the present invention may be the provision of an enantiopure (*R*)-3-hydroxybutyrate acid or a salt thereof. "% identity", as used herein, is obtained by aligning two amino acid sequences to be compared to give a maximum correlation between the sequences. This may include inserting "gaps" in either one or both sequences, to enhance the degree of alignment. A % identity may then be determined over the whole length of each of the sequences being compared. In the above context, an amino acid sequence having a "identity" of at least, for example, 80% to a query amino acid sequence, is intended to mean that the sequence of the subject amino acid sequence is identical to the query sequence except that the subject amino acid sequence may include up to twenty amino acid alterations per each 100 amino acids of the query amino acid sequence. In other words, to obtain an amino acid sequence having a sequence of at least 80% identity to a query amino acid sequence, up to 20% (20 of 100) of the amino acid residues in the subject sequence may be inserted or substituted with another amino acid or deleted. Methods for comparing the identity of two or even more sequences are well known in the art. The percentage to which two sequences are identical can for example be determined by using a mathematical algorithm. Such algorithms are integrated in the BLAST family of programs, e.g. BLAST or NBLAST program, accessible through the home page of the NCBI (www.ncbi.nlm.nih.gov).

The term "motif", as used herein, means a set of amino acids at specific positions along an aligned sequence of evolutionarily related proteins. Amino acids at specific positions are essential in the structure, the stability, or the activity of a protein.

Polyhydroxybutyrate depolymerases can be produced by microbial strains that are widespread in nature. In particular, microbial polyhydroxybutyrate depolymerases can either be located intracellularly or are secreted into the outside of the cells. Especially the cultivation of microbial strains which secrete polyhydroxybutyrate depolymerases provides a simple natural source for manufacturing enzyme preparations exhibiting depolymerase activity. It might be beneficial to induce biosynthesis of the polyhydroxybutyrate depolymerases in such natural strains as it makes sense to the organisms to only produce the enzyme when PHB or a related polymer is available as a nutrient source. Induction of polyhydroxybutyrate depolymerases biosynthesis can for example be performed by addition of PHB or 3-hydroxybutyrate salts to the cultivation medium.

According to another preferred embodiment of the present invention the polyhydroxybutyrate depolymerase is derived from a bacterium of the family *Comamonadaceae*, preferably of the genus Acidovorax, Delftia, Diaphorobacter, Comamonas, Rhodoferax, Caenimonas, Polaromonas, Ramlibacter, Giesbergeria, Xylophilus, Aquabacterium, Ideonella or Variovorax.

According to another preferred embodiment of the present invention the polyhydroxybutyrate depolymerase is derived from Acidovorax sp. BT293, Delftia acidovorans, Acidovorax sp. TP4, Acidovorax sp. KKS102, Acidovorax sp. BE320, Acidovorax sp. ST3, Acidovorax sp. HMWF01 or Acidovorax sp. Root568.

Further, the three-dimensional polymer structure comprising polyhydroxybutyrate (PHB) or a copolymer thereof can be contacted with at least one polyhydroxybutyrate depolymerase as described above and/or with at least one second polyhydroxybutyrate depolymerase. The at least one second polyhydroxybutyrate depolymerases of the present invention is characterized by having at least 80% identity to SEQ ID No. 2 and a motif comprising an amino acid sequence ANLQNSKVYL (SEQ ID No. 5). Preferably, the at least one first and/or the at least one second polyhydroxybutyrate depolymerase comprises a further motif comprising amino acid sequence GATANSMAIG SEQ ID No. 6, or SGATANSMAIGW (SEQ ID No. 7).

The storage stable polymer substrate-enzyme complex of the present invention is obtained by contacting the three-dimensional polymer with at least one polyhydroxybutyrate degrading enzyme at a defined weight ratio between the three-dimensional polymer structure and the at least one polyhydroxybutyrate degrading enzyme. This ratio may be one factor which influences the degrading rate of three-dimensional polymer structure. If more enzyme is added the polymer, it will be degraded faster than in a composition where the enzymes is present in a lower amount. According to a preferred embodiment of the present invention, the weight ratio between the three-dimensional polymer structure and the at least one polyhydroxybutyrate degrading enzyme is preferably 100000000000:1 to 10:1, more preferably 100000000:1 to 10:1, more preferably 1000000:1 to 10:1, more preferably 1000000:1 to 100:1, more preferably 10000000:1 to 1000:1.

After contacting the three-dimensional polymer structure and the at least one polyhydroxybutyrate degrading enzyme, the enzymatic activity of the enzyme is reversibly decreased to obtain a storage stable complex. Since the enzymatic activity is reversibly decreased, degradation of the polymer is blocked and thus, the complex can be stably stored under various conditions. Different methods can be applied to reversibly decrease the enzymatic activity of the at least one polyhydroxybutyrate degrading enzyme.

According to a preferred embodiment of the present invention the enzymatic activity of the at least one poly-hydroxybutyrate degrading enzyme is reversibly decreased by drying, cooling or freezing the polymer substrate-enzyme complex, or by adding a substance comprising at least one enzyme inhibitor to the polymer substrate-enzyme complex.

In particular, drying of the three-dimensional polymer structure by removal of the water is advantageous because it allows obtaining a storage stable complex since the enzymatic activity of the enzymes in the complex is reversibly decreased during storage and can be restored after adding water or an aqueous solution. According to another preferred embodiment of the present invention the polymer substrate-enzyme complex can be dried by removing solvents comprised in the mixture comprising the three-dimensional polymer structure and the depolymerizing enzyme in the solvent. Drying may comprise lyophilization, evaporation or a combination thereof

The enzymatic activity of the at least one poly-hydroxybutyrate degrading enzyme can be reversibly decreased by cooling the polymer substrate-enzyme complex at a temperature between 10°C to 0°C preferably between 8°C to 2°C. The enzymatic activity of the at least one poly-hydroxybutyrate degrading enzyme can also be reversibly decreased by freezing the three-dimensional polymer structure to a temperature between -80°C to 0°C, preferably between -40°C to 0°C, more preferably between -20°C to 0°C.

A substance comprising at least one enzyme inhibitor can also be added to the polymer substrate-enzyme complex to reversibly decrease the enzymatic activity of the at least one polyhydroxybutyrate degrading enzyme. An "enzyme inhibitor", as used herein refers to at least one molecule that binds to an enzyme and blocks its activity. An enzyme inhibitor blocks the enzymatic activity either by binding to the enzyme's active site (thus preventing the substrate itself from binding) or by binding to another site on the enzyme such that the enzyme's catalysis of the reaction is blocked. Enzyme inhibitors may bind reversibly or irreversibly. Irreversible inhibitors form a chemical bond with the enzyme such that the enzyme is inhibited until the chemical bond is broken. By contrast, reversible inhibitors bind non-covalently and may spontaneously leave the enzyme, allowing the enzyme to resume its function. Preferably, reversible inhibitors are used to reversibly decrease the enzymatic activity. According to another preferred embodiment of the present invention the at least one enzymatic inhibitor is a serine hydrolase inhibitor.

The controlled and continuous degradation of the polymer into its monomers and/or oligomers can be initiated by modifying the conditions for reversibly decreasing the enzyme activity of the polymer degrading enzyme to conditions suited for restoring the activity of the enzyme. Suited conditions for restoring the enzymatic activity of the enzyme can be created by adding water or an aqueous solution to the polymer substrate-enzyme complex, by heating or thawing the complex, or by removing the at least one enzymatic inhibitor from the complex. Preferably, the polymer substrate-enzyme complex is heated or thawed to a temperature between 4°C to 40°C, preferably at least 10°C to 40°C, preferably at least 20°C to 40°C, preferably at least 30°C to 40°C to increase the enzymatic activity of the polymer degrading enzyme.

Preferably, the at least one enzymatic inhibitor can be removed from the complex by the degradation of said inhibitor. More preferably, the at least one enzymatic inhibitor can be removed by extraction with a solvent. After degradation or extraction of the at least one enzymatic inhibitor, the enzymatic activity of the bound enzyme is increased and degradation of the polymer into its monomers and/or oligomers can be initiated.

In order to stabilize the at least one polyhydroxybutyrate degrading enzyme and/or to promote its enzymatic activity several steps can be undertaken. Apart from the fact that care should be taken during the preparation of the complex of the present invention (controlled extraction and processing temperatures, minimized processing times, avoidance of processes such as vigorous agitation or the addition of chemicals known to promote protein denaturation) various substances known to the skilled artisan can be added. For instance, the complex may comprise buffering salts to set a specific pH value in which the enzymes are maximally stable. Furthermore, proteolytic inhibitors, reducing agents, glycerol, various carbohydrates, polymers such as polyethylene glycol, amino acids such as glycine, alanine, lysine or threonine, and bulk proteins such as bovine serum albumin (BSA) can be used.

According to another preferred embodiment of the present invention, at least one pharmaceutical active ingredient is comprised within the three-dimensional polymer structure. The pharmaceutically active ingredient can be added to the enzyme/polymer mixture during the manufacturing of the complex.

A "pharmaceutically active ingredient", as used herein, refers to any compound or substance which has a beneficial biological effect on the organism to which it is administered. Such compounds can be inorganic (such as nutritive minerals, trace elements, etc.) or organic compounds, proteins, peptides, nucleic acid molecules etc.

According to a preferred embodiment of the present invention the pharmaceutically active ingredient is preferably selected from the group consisting of small organic molecules (e.g. antibiotics, anti-cancer drugs, pain killers, analgetics, etc.), macromolecules/proteins (e.g. enzymes, biopharmaceuticals, antibodies, etc.), inorganic compounds (e.g. nutritive minerals, trace elements, metal colloids, inorganic nanobodies etc.).

According to another preferred embodiment, after step c) as described above, the complex is coated with a water soluble substance. The coating of polymer-substrate enzyme complex following the contacting the enzyme to the polymer is advantageous because it allows obtaining a composition which can be stored over a long period of time since the enzymes in the composition can be protected from external influences that affect enzyme activity. The enzyme can be activated after adding water or an aqueous solution.

According to a further preferred embodiment, the water soluble substance is selected from the group consisting of water soluble polymers such as gelatin, soluble cellulose derivatives, a variety of further possibilities are described in recent reviews (A Comprehensive Review on Pharmaceutical Film Coating: Past, Present, and Future. Drug Design, Development and Therapy 2020:14 4613-4623, doi:10.2147/DDDT.S277439; Pharmaceutical Application of Tablet Film Coating Pharmaceutics 2020, 12, 853; doi:10.3390/pharmaceutics12090853)

According to another preferred embodiment, after step c) as described above, the complex is coated with an enteric coating. The coating of the polymer-substrate enzyme complex can be carried out after contacting the enzyme with the polymer.

Enteric coatings enclose pharmaceutical preparations which comprise acid sensitive pharmaceutically active compounds or whose active compounds shall be released in the intestine and not in the stomach. Typically, enteric coated dosage forms include tablets or capsules comprising the polymer substrate-enzyme complex of the present invention. Any enteric coating should be applied to a sufficient thickness such that the entire coating does not dissolve in the gastrointestinal fluids at pH below about 5, but does dissolve at pH about 5 and above. It is expected that any anionic polymer exhibiting a pH-dependent solubility profile can be used as an enteric coating in the methods and compositions described herein to achieve delivery to the lower gastrointestinal tract. Shellac, also called purified lac, a refined product obtained from the resinous secretion of an insect is often used in enteric coatings and dissolves at a pH>7. Alternatively, also acrylic polymers and cellulose derivatives can be used in enteric coatings. Examples of suitable acrylic polymers include methacrylic acid copolymers and ammonium methacrylate copolymers. The Eudragit series E, L, S, RL, RS and NE (Rohm Pharma) are available as solubilized in organic solvent, aqueous dispersion or dry powders. The Eudragit series RL, NE, and RS are insoluble in the gastrointestinal tract but are permeable and are used primarily for colonic targeting. The Eudragit series E dissolve in the stomach. The Eudragit series L, L-30D and S are insoluble in stomach and dissolve in the intestine. Suitable cellulose derivatives include ethyl cellulose and reaction mixtures of partial acetate esters of cellulose with phthalic anhydride, in particular cellulose acetate phthalate, cellulose acetate trimellitate (Eastman), methylcellulose (Pharmacoat, Methocel), hydroxypropylmethyl cellulose phthalate (HPMCP), hydroxypropylmethyl cellulose succinate (HPMCS) and hydroxypropylmethylcellulose acetate succinate. The enteric coating used herein can contain further plasticizers, colorants, talc, and magnesium stearate, which are well known in the art. Examples of plasticizers include triethyl citrate, triacetin (glyceryl triacetate), acetyl triethyl citrate, Carbowax 400 (polyethylene glycol 400), diethyl phthalate, tributyl citrate, acetylated monoglycerides, glycerol, fatty acid esters, propylene glycol and dibutyl phthalate.

Another aspect of the present invention relates to a storage stable polymer substrate-enzyme complex obtainable by the method as described herein. The complex of the present invention may comprise a three-dimensional polymer structure and at least one polyhydroxybutyrate polymer degrading enzyme attached to said polymer, wherein said polymer comprises polyhydroxybutyrate (PHB) or a copolymer thereof. The complex of the present invention may comprise more than one three-dimensional polymer structure and/or more than one polyhydroxybutyrate degrading enzyme. In a preferred embodiment of the present invention the complex of the present invention may comprise at least two, more preferably at least three, more preferably at least four, more preferably at least five, three-dimensional polymer structures and/or polyhydroxybutyrate degrading enzymes.

According to another preferred embodiment, the complex of the present invention may comprise polyhydroxybutyrate (PHB), wherein the polyhydroxybutyrate is selected from the group consisting of poly-3-hydroxybutyrate and poly-4-hydroxybutyrate. Preferably, the copolymer is selected from the group consisting of poly(3-hydroxybutyrate-co-3-hydroxyvalerate).

According to a further preferred embodiment of the present invention, the at least one polyhydroxybutyrate degrading enzyme can be a polyhydroxyalkanoate depolymerase, preferably a polyhydroxyalkanoate depolymerase derived from bacteria, archaea, or fungi.

According to another preferred embodiment of the present invention the polymer substrate-enzyme complex may comprise a three-dimensional polymer structure comprising polyhydroxybutyrate and at least one polyhydroxybutyrate degrading enzyme attached to the surface of said polymer structure.

According to another preferred embodiment, the complex of the present invention may comprise at least one polyhydroxybutyrate degrading enzyme comprising a polyhydroxybutyrate (PHB) binding domain.

According to a further preferred embodiment of the present invention, the polyhydroxybutyrate degrading enzyme is selected from the group consisting of hydrolases, esterases, polyesterases, polyester depolymerases, polyhydroxyalkanoate depolymerase, polyhydroxybutyrate depolymerase and combinations thereof.

According to a very preferred embodiment of the present invention the complex may comprise objects consisting of at least one three-dimensional polymer structure and at least one associated polyhydroxybutyrate degrading enzyme attached to its surface which can be stored and will be degraded upon contact with water. The ratio of enzyme attached to the surface enhanced polymer can be variable and determines the degradation velocity. Thus, by the enzyme to polymer ratio the degradation velocity can be triggered. The polymer substrate-enzyme complex of the present invention is further characterized that the weight ratio between the three-dimensional polymer structure and the at least one polyhydroxybutyrate degrading enzyme is preferably 100000000000:1 to 10:1, more preferably 100000000:1 to 10:1, more preferably 1000000:1 to 10:1, more preferably 1000000:1 to 100:1, more preferably 10000000:1 to 1000:1.

The attached at least one polyhydroxybutyrate degrading enzyme can stay active when bound to the three-dimensional polymer structure under chilled, frozen, moist or dry conditions.

According to another further embodiment of the present invention, the complex can be stored for at least 10 days, preferably for at least 100 days, more preferably for at least 12 months, more preferably for at least two years.

According to another further embodiment of the present invention, the complex can be stored at a temperature between - 80°C to 40°C, preferably between -20°C to 40°C, more preferably between 0°C to 40°C, more preferably between 4°C and 40°C.

According to another further embodiment of the present invention the complex may be coated with a water soluble substance.

According to another preferred embodiment of the present invention the complex may be coated with an enteric coating.

According to another further embodiment of the present invention, the complex may comprise pharmaceutical active ingredients. The composition of the present invention may comprise additionally to the other components at least one, preferably at least two, more preferably at least three, more preferably at least five, more preferably at least ten, pharmaceutical active ingredient which are released in the course of the degradation of the three-dimensional polymer structure. This allows using the composition of the present invention as a controlled release composition. The release rate of e.g., pharmaceutically active ingredients can be influenced by the ratio between the three-dimensional polymer structure and the at least one polyhydroxybutyrate degrading enzyme degrading enzyme as specified above.

According to another preferred embodiment of the present invention the complex can be provided as a food or feed supplement. It turned out that 3-hydroxybutyrate can be provided in the correct enantiomeric (*R*)form when PHB is hydrolyzed by at least one polyhydroxybutyrate depolymerase of the present invention in the form of a food supplement. 3-hydroxybutyrate is of great interest as a keto nutrient. The complex may comprise at least one three-dimensional polymer structure comprising PHB or a copolymer thereof and at least one associated polyhydroxybutyrate degrading enzyme, preferably the polyhydroxy butyrate depolymerase as described above, attached to its surface which can be stored under dry conditions and will be degraded into enantiopure (*R*)-3-hydroxybutyrate monomers upon contact with water. The complex can be enveloped in capsules or coatings which are resistant to the stomach and get dissolved and released into the aqueous environment of the intestine. As the pH in the intestine is slightly alkaline and thus well suited for the action of the enzyme, the (*R*)-3-hydroxybutyrate monomers can get released, absorbed, and further metabolized. The benefit of this strategy in comparison to a direct application of the monomer as nutrient is, that the release of the (*R*)-3-hydroxybutyrate from the polymer is in a slow manner and thus peaks of higher concentrations are avoided. Encapsulation can either be performed by standard acid-resistant capsules or in a micro-coating process.

Another aspect of the invention relates to the use of a storage stable polymer substrate-enzyme complex as an or as a part of an implant and/or as carrier or scaffold in human and/or animal cell culture. In particular, applications include processes to employ animal or human cells or with cultivation of structured cell or tissue layers, organoids, or larger organ parts. Further applications are with the production of artificial meat based on cultivation of animal cells or tissues on scaffolds based on the complex of the present invention.

Other forms of the complex of the present invention are implants to replace a missing biological structure, support a damaged biological structure or enhance an existing biological structure. Commonly known and used implants are introduced into an animal or human body via surgery and removed after a certain period of time by a further surgical intervention. The use of the complex of the present invention to manufacture such implants has the advantage that the implants are removed from the body by a degradation process catalyzed by the at least one polyhydroxybutyrate degrading enzyme. Therefore, a further surgical intervention to remove the implant is no longer necessary. Typical implants comprising or consisting of the composition of the present invention are pins, rods, screws and plates used to anchor fractured bones, for instance.

Implants may also serve, for instance, as a depot of pharmaceutically active substances which are released during degradation of the polymer within the body. These drug releasing properties may also be used to coat implants made of titan or other non-degradable materials. Another preferred embodiment relates to an implant comprising a complex according to the present invention.

According to a preferred embodiment of the present invention the implant may have various forms such as a bone screw, a bone nail or a thread or may be an implant with controlled drug release.

### EXAMPLES

### Example 1: Identification and Isolation of a Polyhydroxybutyrate (PHB) depolymerase (DP) and Construction of an Expression Vector with PHB-DP

To obtain an active depolymerase a gene library of a PHB-degrading bacterium (*Acidovorax sp*.) isolated from soil samples was created. The genomic DNA of the mentioned bacterium from soil samples was isolated and partially restricted by *Sau3A I.* The obtained DNA-fragments were cloned into the screening vector [pZero^{™}-2, ThermoFisher]. This vector was subsequently transformed to *E. coli* TOP10 by electroporation. The clones were plated out on selection agar plates containing suspended PHB powder (10 g/L) and screened for PHB degradation at 37°C (incubation time 12 to 28 h). If a recombinant clone was able to degrade PHB a clear zone appeared where the substrate was degraded.

The colony with the highest degrading activity was isolated and the insert was sequenced. Several open reading frames were detected and one with sequence characteristics of a putative PHB-DP was identified by BLAST searches. This fragment was amplified by PCR and introduced into an expression vector, [pMS470-C termHis] (based on Balzer et al., Nucleic Acids Research, 20(1992):1851-1858). By cloning the fragment into this vector, a His-tag on the C-terminus of the protein was added allowing efficient purification.

The sequenced gene encoding PHB-DP has a subtype B catalytic domain and a SBD (substrate binding domain) type I. All reported catalytic amino acid sequences such as the oxyanion-hole as well as a catalytic aspartic and a catalytic histidine are present in this protein. The enzyme has not yet been described in literature but shares 99% amino acid identity with a protein of *Acidovorax sp.* KKS102 (NCBI Reference Sequence: WP_015014950.1).

The PHB-DP identified has the following amino acid sequence (SEQ ID No. 1):

MAFNFIRAAAAGAAMALCGAGGVHAAVNLPALKIDKTQTTVSGLSSGGFMAVQL
HVAYSATFAKGAGVVAGGPFYCAEGSIVNATGRCMASPAGIPTSTLVSTTNTWA
SQGVIDPVANLQTSKVYLFSGTLDSVVKTGVMDALRTYYNSFVPAANVVYKKDI
AAEHAMVTDDYGNACSTKGAPYISDCNFDLAGAMLQHLYGTLNARNNATLPTGN
YIEFNQSEFITNHGMATTGWAYVPQACQAGGTATCKLHVVLHGCKQNISDVQQQ
YVRNTGYNRWADTNNIVMLYPQTSTAATNSCWDWWGYDSANYSKKSGPQMAAIK
AMVDRVSSGTGGTTPPDPVALPAPTGVSTSGATASSMAIGWAAVTGAASYNVYR
NANKVNALPVTATSYTDTGLAASTTYSWTVRAADANGAEGATSAAASGTTLAAS
GGGTATCTTASNYAHTLAGRAYAAGGYTYALGSNQNMGLWNVFVTNTLKQTSTN
YYVIGTCP

According to the analysis of the amino acid sequence with the SignalP program (Petersen et al.,Nature Methods, 8:785-786, 2011)the protein contains a signal peptide which gets processed by proteolytic cleavage during the export from the cell into the external space. The resulting mature protein has thus the following amino acid sequence (SEQ ID No. 2).

AVNLPALKIDKTQTTVSGLSSGGFMAVQLHVAYSATFAKGAGVVAGGPFYCAEG
SIVNATGRCMASPAGIPTSTLVSTTNTWASQGVIDPVANLQTSKVYLFSGTLDS
VVKTGVMDALRTYYNSFVPAANVVYKKDIAAEHAMVTDDYGNACSTKGAPYISD
CNFDLAGAMLQHLYGTLNARNNATLPTGNYIEFNQSEFITNHGMATTGWAYVPQ
ACQAGGTATCKLHVVLHGCKQNISDVQQYVRNTGYNRWADTNNIVMLYPQTST
AATNSCWDWWGYDSANYSKKSGPQMAAIKAMVDRVSSGTGGTTPPDPVALPAPT
GVSTSGATASSMAIGWAAVTGAASYNVYRNANKVNALPVTATSYTDTGLAASTT

YSWTVRAADANGAEGATSAAASGTTLAASGGGTATCTTASNYAHTLAGRAYAAG
GYTYALGSNQNMGLWNVFVTNTLKQTSTNYYVIGTCP

Both, the unprocessed and processed forms are enzymatically active.

The PHB-DP containing a His-Tag at the C-terminus to facilitate purification has the following amino acid sequence (SEQ ID No. 3, the signal sequence is underlined and put in parentheses as it will be not present in the processed protein, a short linker sequence between the coding sequence and the his-tag is marked by italicized letters):

MAFNFIRAAAAGAAMALCGAGGVHAAVNLPALKIDKTQTTVSGLSSGGFMAVQL
HVAYSATFAKGAGVVAGGPFYCAEGSIVNATGRCMASPAGIPTSTLVSTTNTWA
SQGVIDPVANLQTSKVYLFSGTLDSVVKTGVMDALRTYYNSFVPAANVVYKKDI
AAEHAMVTDDYGNACSTKGAPYISDCNFDLAGAMLQHLYGTLNARNNATLPTGN
YIEFNQSEFITNHGMATTGWAYVPQACQAGGTATCKLHVVLHGCKQNISDVQQQ
YVRNTGYNRWADTNNIVMLYPQTSTAATNSCWDWWGYDSANYSKKSGPQMAAIK
AMVDRVSSGTGGTTPPDPVALPAPTGVSTSGATASSMAIGWAAVTGAASYNVYR
NANKVNALPVTATSYTDTGLAASTTYSWTVRAADANGAEGATSAAASGTTLAAS
GGGTATCTTASNYAHTLAGRAYAAGGYTYALGSNQNMGLWNVFVTNTLKQTSTN
YYVIGTCPKLAAALEHHHHHH*

### Example 2: Expression and purification

Expression was performed in *E. coli* XL1 by cultivation over 24h without induction by IPTG. The fermentation broth was centrifuged, the cell pellet washed and resuspended in 100 mM potassium phosphate buffer, pH 7.4 and cells were disrupted by ultrasonic treatment. The cell debris and insoluble components were removed by centrifugation (20,000 x g) and the supernatant sterile filtered (0.2 pm) prior protein purification. This supernatant contains intracellular and periplasmic proteins and thus contains both processed and unprocessed PHB-DP protein. Protein purification was performed according the standard protocol for His-tagged proteins (GE Healthcare, Ni Sepharose FastFlow). The buffer containing imidazole was exchanged directly after purification against 100 mM potassium phosphate buffer, pH 7.4 by gel filtration.

### Example 3: Activity Assay

10 mg of PHB powder (Enmat Y3000, TianAn Biopolymer, Ningbo City, China) were placed in a 2 mL polypropylene tube and mixed with 1 mL enzyme solution in 200 mM potassium phosphate buffer, pH 7.4. The samples were incubated at 25°C in a shaking incubator (550 rpm. Samples (40 pL) were taken in time intervals, centrifuged at 10,000 x g, the supernatants collected and kept frozen at -20°C until further use. For determination of activity, the frozen samples were thawed and the amount of released 3-HB was determined by an enzymatic method employing a ready to use reagent kit (Megazyme, Product code K-HDBA, www.megazyme.com).

### Example 4: Preparation of a biodegradable polymer composition from polymer dissolved in a solvent

To prove the ability of degrading PHB-films in liquid buffer, PHB was dissolved in chloroform at 10% w/v and a bromophenol blue (BPB) stock solution was added (1% v/v) as an easily traceable model compound. Furthermore, the same polymer films comprising in addition PHB depolymerase enzyme were prepared. The PHB depolymerase was added to the CHCl₃ dissolved PHB either by adding 0,01 mg lyophilized enzyme powder or by adding 50 µL of an aqueous enzyme stock solution (0,2 mg/mL).

For preparation of integrated polymer matrix films 500 µL of these solutions were pipetted on a glass plate and dried in a fume hood at room temperature.

Degradation assays were performed in 5 mL of MSM in 50 mL Greiner tubes at 37°C for 24 and 48 hours.

When the enzyme was degrading the polymer also released the model compound from the polymer and thereby the solution turned blue. Four different samples were performed in parallel:
1. Negative control, PHB/PBP-film without enzyme
2. Positive control, PHB/PBP-film without integrated degrading enzyme and 50 µL enzyme (0.2 mg/mL) added externally directly to the buffer
3. PHB/BPB-film with integrated lyophilized degrading enzyme
4. PHB/BPB-film with integrated enzyme added as aqueous liquid stock solution

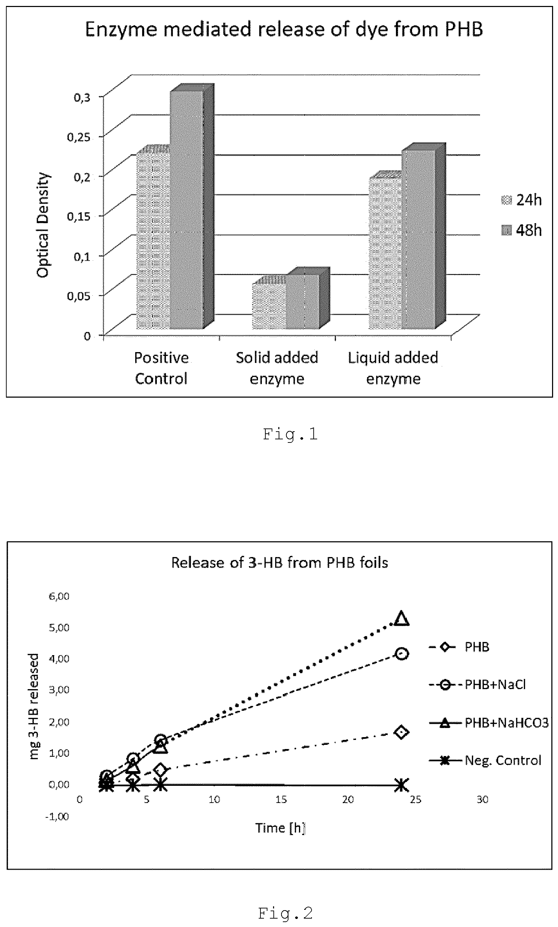

The release of the model compound (blue color) was measured photometrically. As shown in Fig. 1 the dye is released to the buffer in every sample except the negative control without enzyme. This provides a clear proof that
a) the PHB polymer gets degraded by the action of a PHB depolymerase integrated into the polymer matrix and
b) a compound in addition integrated in a PHB polymer/depolymerase matrix can be released by the degradative action of the enzyme.

### Example 5: Creation of a surface enhanced hydrophobic polymer by inclusion of water-soluble particles.

PHB powder (Enmat Y3000, TianAn Biopolymer, Ningbo City, China) was mixed with sodium chloride or sodium hydrogen carbonate powder. The mixtures were used to prepare polymer-salt compositions by producing thin sheets using a thermo-hydraulic press (Graspresso GP6003, Steler GmbH, Marzling, Germany). Detailed description of the experiment:
0.1 g PHB powder containing 20 % NaCl or 5% NaHCO₃ powder were spotted on a sheet of household aluminum foil at a size of about 15 x 15 mm. This sheet was put into the press which was preheated to 155°C and covered with a second aluminum sheet. Then the pressure was applied for 20 seconds at maximum capacity (about 9 tons according to the specification of the manufacturer). After the pressing period the press was immediately opened and the aluminum foils with the included pressed foil removed and cooled down at room temperature for 5 min. The pressed PHB sheet was then removed from the aluminum foils and stored in a plastic beaker. The same procedure was performed with 0.1 g PHB (without added salt) in order to produce a PHB sheet without salt load. The obtained PHB sheets were weighted and the washed with distilled water at 60°C for 24 h. The NaCl and NaHCO₃ loaded sheets had weight losses of 21% and 2.4%, respectively, no weight loss was measured with the non-loaded sheet.

Pieces of 10 x 10 mm were cut of these produced PHB foils (loaded with 20% NaCl or 5% NaHCO₃, and as control unloaded) and weighted. For preparation of a composition of polymer and polymer degrading enzyme these sheets were incubated in 2 mL of 200 mM potassium phosphate buffer (pH 7.5) containing 0.5 % (w/w) purified PHB depolymerase (PHB-DP, according to examples 1 and 2) for 30 min at 4°C.

Then the samples were incubated at 25°C for up to 24 h. Samples were taken at 2, 4, 6, and 24 h and the amount of released 3-hydroxybutyrate monomer determined by an enzymatic method using a ready to use reagent kit (Megazyme Product code K-HDBA, www.megazyme.com).

The results shown in Fig. 2 clearly demonstrate that enhancing the surface by creating pores through dissolving salt particles included in the polymer matrix enhances the degradation velocity.

The treated PHB sheets were removed from the incubation buffer after 24 h treatment, washed with distilled water, dried and weighted. The control sheet did not show any weight loss, the sheets without NaCl and with NaCl had weight losses of 7.9% and 23.6%, respectively, which is in good correlation with the data shown in Fig.2.

### Example 6: Creation of a surface enhanced hydrophobic polymer object by electrospinning and degradation by PHB-DP

Electrospun PHB sheets were performed by dissolving PHB (same as in example 5) in chloroform-based solvents. Three different setups were used:

| Sheets | Solution composition | Concentration | Feeding rate | voltage | Thickness (µm) |
|---|---|---|---|---|---|
| Sheet #1 | 15 g PHB + 285 g chloroform + 120 dichloroethane + 30 g DMF | 3.3 wt% | 5 mL/h | 18 kV | 68 ± 2 |
| Sheet #2 | 15 g PHB + 285 g chloroform + 120 dichloroethane + 30 g DMF | 3.3 wt% | 5 mL/h | 18 kV | 105 ± 7 |

| | | | | | |
|---|---|---|---|---|---|
| Sheet #3 | 10 g PHB + 100 g chloroform + 20 g acetonitrile | 7.7 wt% | 10 mL/h | 20 kV | 85 ± 2 |

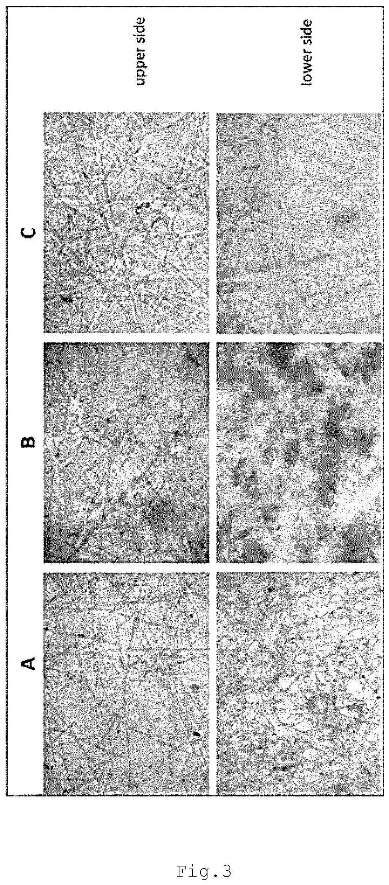

Fig.3 shows microscopic analysis of the obtained structures. Sheet #3 (C) showed the best network of PHB fibers, sheets #1 (A) and #2 (B) were not a clear network of fibers, at the lower side the fibers were more or less molten together.

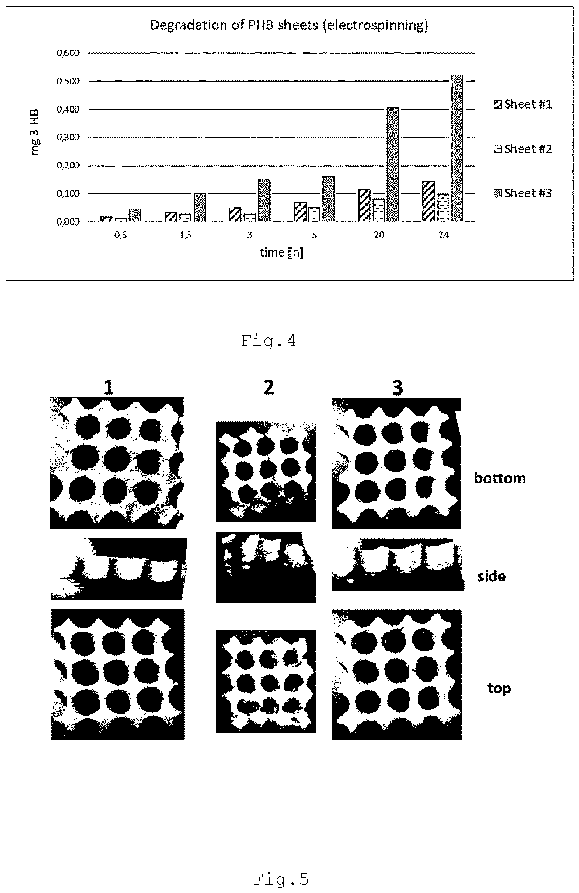

Pieces of 10 x 10 mm were cut form these sheets and washed with distilled at 60°C for 24 h, then dried for 3h at 60°C and weighted. Thereafter, the samples were loaded with 0.1% or 0.05% (w/w PHB) PHB-DP in 1 mL 200 mM potassium phosphate buffer (pH 7.4) by incubation at 4°C for 10 min and shaking at 300 rpm. Thereafter the samples were removed from the PHB-DP/buffer solution, rinsed with buffer, frozen at -80°C for 3 h and freeze-dried. The freeze-dried samples were then incubated in 1 mL 200 mM potassium phosphate buffer (pH 7.4) and incubated at 25°C and 550 rpm on a shaker. Samples were taken in time intervals and the amount of release 3-HB was determined by the enzymatic method as described in example 5. The results shown in Fig.4 clearly demonstrate that the sheet #3 showing the best network of fibers has the highest degradation (release of 3-HB) velocity. Pieces of all 3 types of the sheets not loaded with PHB-DP were also incubated with buffer as control and did not show significant release of 3-HB.

### Example 7: Creation of a surface enhanced hydrophobic polymer by laser sintering and association of PHB-DP.

PHB powder (see example 5) was fractionated using sieves (Sieve device Retsch AS200) and fractions 100 µm < x < 200 µm were recovered. For improvement of the coating behavior Aerosil^{®} was added.
Laser sintering was conducted using the following conditions:
Process Temperature:165°C
Laser Power: 19 W
Scanning Speed: 3 m/sec
Hatch distance: 250 µm
Layer distance: 300 µm

Number of layers: 30 (Lot #1) and 35 (Lots #2 & #3) The encircled part at lot #1 bottom view indicates the pieces taken for creation of enzyme loaded samples. 3 lots of sintered objects were prepared. The resulting objects shown in Fig.5 demonstrate that it is possible to create porous structures with this technology. It was recognized that with lot #2 (smaller total size) the consistency was rather weak indicating insufficient sintering. Lots #1 and #3 had a sufficient stability.

For creation of samples loaded with PHB-DP pieces of these created objects were cut at approximately same parts as indicated in Fig.5 and weighted. The pieces from lots #1 had weights between 211 mg and 240 mg; The pieces of lot #2 had weights between 46 mg and 79 mg and the pieces of lot 3 had weights between 280 mg and 338 mg.

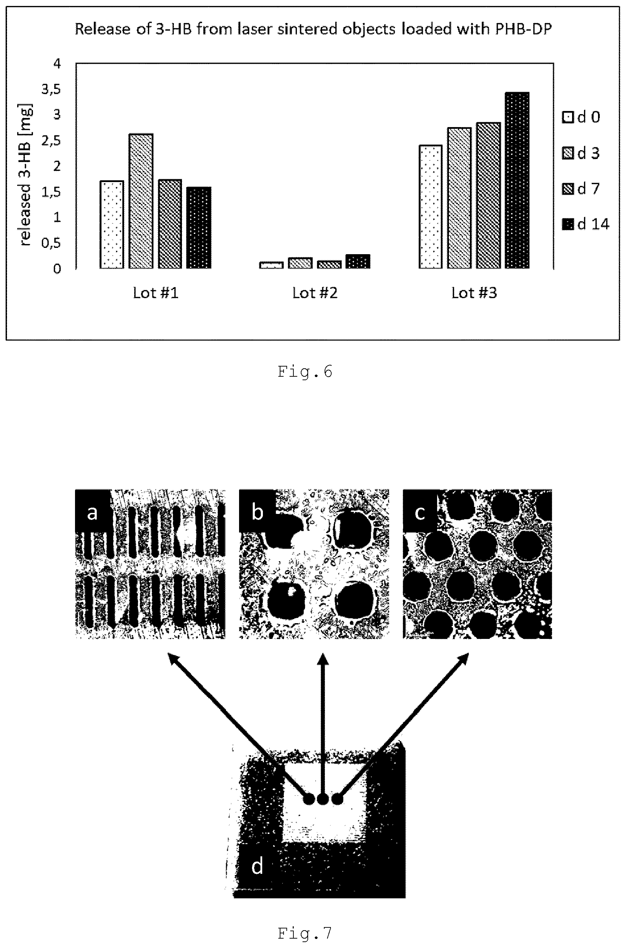

Samples were loaded with 0.06% PHB-DP (w/w) by incubation in 2 mL potassium phosphate buffer (pH 7.4) containing the appropriate amount of PHB-DP for 10 min at 4°C and shaking at 550 rpm. The samples were then taken out and frozen at -80°C for about 1.5 h, lyophilized and stored at 4 °C for 0, 3, 7 and 14 days. The samples were analyzed after different times of storage for release of 3-HB by incubation in 2 mL potassium phosphate buffer (pH 7.4) for 5 h. The amount of released 3-HB was determined by the enzymatic method as described in example 5. The results shown in Fig. 6 demonstrate that the activity of the loaded PHB-DP is maintained over the storage time. The samples from lot #2 only showed low release of 3-HB. On the one hand the pieces of lot 2 were smaller and on the other hand the consistency was rather bad.

### Example 8: Creation of a surface enhanced hydrophobic polymer by laser mediated surface structuring

Cubic PHB objects of the dimension 20 x 20 x 2 mm were prepared by melting PHB powder at 176°C and slightly pressing the material in an appropriate form. After cooling the smooth surface was treated by laser to create enhanced surfaces by burning defined structures into the polymer within an area of 10 x 10 mm. 3 different types of structures were generated in a distance of 100 pm:
a) Linear lamellar cavities
b) Pyramid-shaped cavities
c) Cylindrical cavities in a hexagonal arrangement.

The depth of the cavities was set to 100, 200 or 500 µm in different series.

The setup of the laser system included a Fuego laser (Lumentum Operations LLC), a Scanner (IntelliScan, Scanlab) and a S4LFT4010/075 optics system (SillOptics GmbH) with a focal length of 100 mm and a spot size of 15 µm. The laser wavelength was 355 nm; an average power of 3.5 W and a pulse period of 10 ps at a pulse frequency of 5 kHz were applied. The feed speed was 25 mm/s.

Fig. 7 shows pictures of the PHB objects generated with the different structures.

### Example 9: Creation of a surface enhanced hydrophobic polymer by thermo-mechanical structuring.

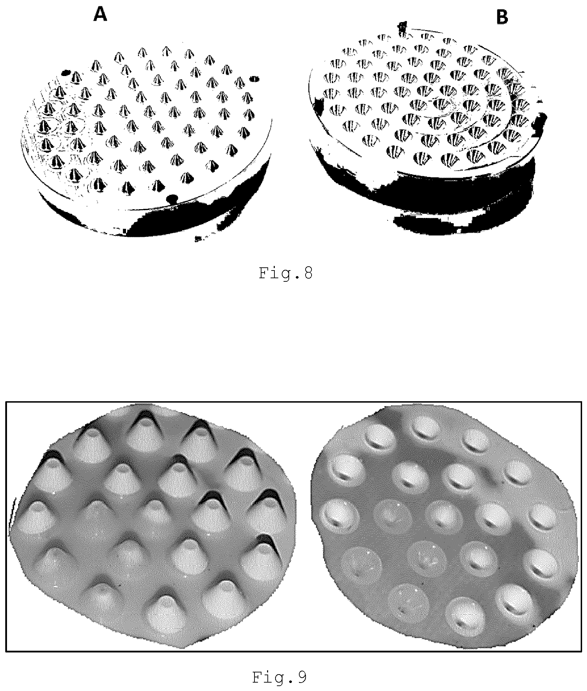

A special model consisting of a "negative" and a "positive" unit was constructed made of aluminum by a computer guided mortising machine. The positive unit hat cones and the negative units corresponding cone-shaped holes, fitting together with a distance between the cones and the holes of 0.2 mm. Fig.8 shows a picture of the model units.
The negative part of the model was coated with 500 mg PHB powder (same as in Example 5) within the elevated outer ring and the positive unit was placed on top. The combined unit was placed in the thermo-hydraulic press (Graspresso, see example 5) without applying pressure and heated to 155°C. When this temperature was reached the pressure of about 9 tons was applied for 60 seconds and the heating of the press was shut off allowing the temperatures to slowly decrease to room temperature. Thereafter the two parts of the model were separated and the PHB object was removed. In Fig.9 the resulting structure is shown.

### Example 10: Associating a polymer degrading enzyme to a surface enhanced hydrophobic polymer by adsorption of the enzyme from aqueous solution and long time storage.

2.5 g PHB powder (see example 3) were finely grounded in a mortar with a pestle in order to create a high surface and suspended in a final volume of 18 mL 200 mM potassium phosphate buffer, pH 7.4 containing different amounts (3, 1, 0.3 and 0.1 µg protein per mg PHB) of purified PHB-DP (see example 2). The mixtures were incubated for 10 min at 4°C on a shaker (300 rpm) and then centrifuged (3000 x g). The supernatant was removed and the powder was lyophilized overnight. The dry powder was to one part stored at 4°C and to the other part at room temperature.

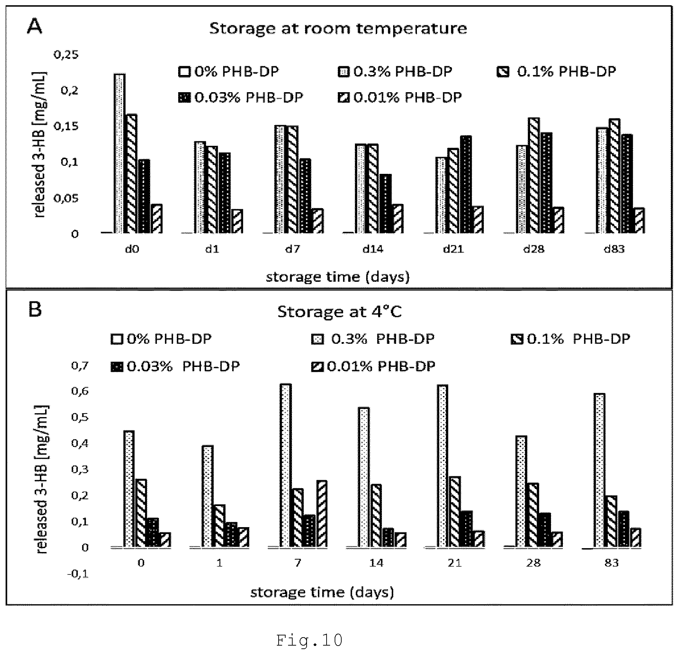

Samples (5 mg) were taken at time intervals, suspended in 500 µL potassium phosphate buffer, pH 7.4 and incubated at 25°C for 10 min. The release of monomeric 3-HB was determined in the solution after removing the PHB by centrifugation using the kit as described in Example 3. The results shown in Fig.10 demonstrate that the PHB-DP enzyme remains associated to the PHB polymer and is stable over a long time period. Storage at 4°C did not show any significant loss of enzyme activity associated to the PHB polymer particles. Storage at room temperature resulted in an initial loss of activity, but then remained more or less constant over the remaining storage time.

### Example 11: Creation of a surface enhanced hydrophobic polymer and simultaneous embedding a pharmaceutical compound in it by employing the thermo-hydraulic pressure method.

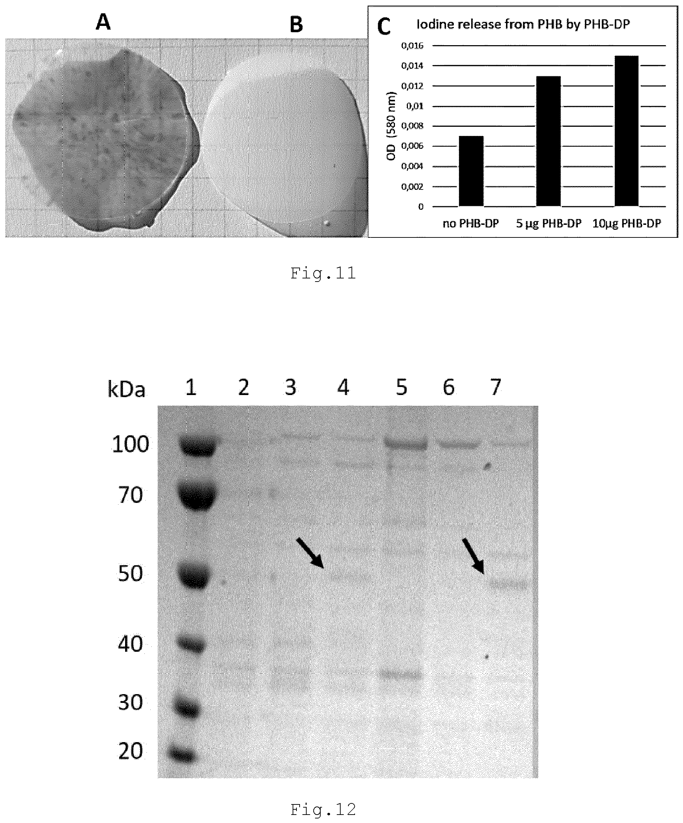

To 1 g of PHB powder (see Example 3) 70 mg of Betaisodona (10% polyvidone-iodine complex, Mundipharma GmbH, Vienna) solution were added and the powder was thoroughly mixed with the liquid by rubbing for about 10 min in a flat beaker using a stainless steel spoon. This resulted in a nearly homogeneous integration of the liquid into the powder indicated by an overall brownish coloring of the powder. 200 mg of this powder was pressed to a sheet by the thermo-hydraulic method as described in example 5. The sheet was washed with 200 mM potassium phosphate buffer, pH 7.4 and dried. Pieces of 10 x 10 mm were cut out and placed in 500 µL PHB-DP solution in 200 mM potassium phosphate buffer, pH 7.4. Two concentrations were used: 10 and 5 µg PHB-DP per mg PHB. For a control only buffer without PHB-DP was used. For a blank a sheet without Betaisodona was used. Following incubation for 4 h, the released iodine was semi-quantitatively determined by the starch complex method (Xiao Z. et al., Analytical Biochemistry, 2006, 351:146-148). The results shown in Fig.11 demonstrate that the iodine was released upon action of the PHB-DP associated to the polymer. Due to the small size of the analyzed sheets only small amounts of iodine were released.

### Example 12: Induction of secretory production of PHB-DP during cultivation of Acidovorax species BT293

The PHB-degrading bacterium Acidovorax species BT293 was inoculated in 30 mL 1:50 diluted LB medium in 100 mL shake flasks. One part of the cultures was induced by addition of 0.1 g/L 3-HB sodium salt, one part was induced by addition one 1 g/L PHB powder (Enmat Y3000, TianAn Biopolymer, Ningbo City, China), a third part was left without adding an inducing substance. The cultures were incubated on a laboratory shaker at 28°C or 37°C for 3 days and the cells removed by centrifugation. 13 µL of the supernatants were denatured under reducing conditions by heating with loading buffer and applied on SDS-polyacrylamide gels. The results summarized in Fig. 12 clearly demonstrate induction of a protein having the size of the PHB-DP in cultures where PHB was added as inducer.

## Claims

1. A method for obtaining a storage stable polymer substrate-enzyme complex comprising:
a) providing a three-dimensional polymer structure comprising polyhydroxybutyrate (PHB) or a copolymer thereof,
b) contacting the three-dimensional polymer structure of step a) with at least one polyhydroxybutyrate degrading enzyme in a solvent to form a three-dimensional polymer structure-enzyme complex, and
c) reversibly decreasing the enzymatic activity of the least one polyhydroxybutyrate degrading enzyme.

2. The method according to claim 1, wherein the polyhydroxybutyrate (PHB)is selected from the group consisting of poly-3-hydroxybutyrate and poly-4-hydroxybutyrate.

3. The method according to claim 1 or 2, wherein the copolymer is poly(3-hydroxybutyrate-co-3-hydroxyvalerate).

4. The method according to any one of claims 1 to 3, wherein the three-dimensional polymer structure is produced by 3-D printing, electrospinning, laser sintering, thermo-mechanical structuring, and combinations thereof.

5. The method according to any of claims 1 to 4, wherein the specific surface area of said three-dimensional polymer structure is increased a chemical, physical, enzymatic method or a combination thereof.

6. The method according to claim 5, wherein the physical method to increase the specific surface area of the three-dimensional polymer structure is selected from the group consisting of milling, drilling, grinding, cutting, embossing, and/or the machining of structures from filaments, tubes, and combinations thereof.

7. The method according to claim 5 or 6, wherein the physical method to increase the specific surface area of the three-dimensional polymer structure results in the formation of pores, holes, folds, channels, wells, and combinations thereof within the shaped polymer substrate.

8. The method according to claim 5, wherein the chemical or enzymatic method to increase the specific surface area of the three-dimensional polymer structure is selected from the group consisting of etching, partial degradation, partial hydrolysis, partial dissolving, partial breaking of polymer strands and combinations thereof

9. The method according to any one of claims 1 to 8, wherein the at least one polyhydroxybutyrate degrading enzyme comprises a polyhydroxybutyrate (PHB) binding domain and is preferably selected from the group consisting of hydrolases, esterases, polyesterases, polyester depolymerases, polyhydroxyalkanoate depolymerase, polyhydroxybutyrate depolymerase and combinations thereof.

10. The method according to any one of claims 1 to 9, wherein the at least one polyhydroxybutyrate degrading enzyme is a polyhydroxyalkanoate depolymerase, preferably a polyhydroxyalkanoate depolymerase derived from bacteria, archaea, or fungi.

11. The method according to any of the claims 1 to 10, wherein at least one pharmaceutical active ingredient is comprised within the three-dimensional polymer structure-enzyme complex.

12. The method according to any of the claims 1 to 11, wherein the enzymatic activity of the at least one poly-hydroxybutyrate degrading enzyme is reversibly decreased by drying, cooling or freezing the polymer substrate-enzyme complex, or by adding a substance comprising at least one enzyme inhibitor to the polymer substrate-enzyme complex.

13. The method according to any of the claims 1 to 12, wherein after step c) the complex is coated with a water soluble substance.

14. A storage stable polymer substrate-enzyme complex obtainable by a method according to any one of claims 1 to 13.

15. A storage stable polymer substrate-enzyme complex according to claim 14 comprising a three-dimensional polymer structure and at least one polyhydroxybutyrate degrading enzyme attached to said polymer, wherein said polymer comprises polyhydroxybutyrate (PHB) or a copolymer thereof.

16. Use of a storage stable polymer substrate-enzyme complex according to claim 14 or 15 as an or as a part of an implant and/or as carrier or scaffold in human and/or animal cell culture.
